(19) Europäisches Patentamt
European Patent Office
Office européen des brevets

(11) EP 0 855 405 B1

(12) EUROPÄISCHE PATENTSCHRIFT

(45) Veröffentlichungstag und Bekanntmachung des Hinweises auf die Patenterteilung:
**19.11.2003 Patentblatt 2003/47**

(51) Int Cl.[7]: **C08B 31/04**, A61L 15/28

(21) Anmeldenummer: **98100933.5**

(22) Anmeldetag: **21.01.1998**

(54) **Verfahren zur Herstellung von quellbaren, alterungsbeständigen Stärkemaleaten, biologisch abbaubare Stärkemaleate sowie Verwendung**

Process for preparing swellable, aging resistant starch maleates: biodegradable starch maleates and their use

Procédé pour la préparation de maléates d'amidon gonflables et résistants au vieillissement, du maléate d'amidon biodégradable et son usage

(84) Benannte Vertragsstaaten:
**AT BE CH DE DK ES FI FR GB GR IE IT LI NL PT SE**

(30) Priorität: **25.01.1997 DE 19702641**

(43) Veröffentlichungstag der Anmeldung:
**29.07.1998 Patentblatt 1998/31**

(73) Patentinhaber: **Stockhausen GmbH & Co. KG
47805 Krefeld (DE)**

(72) Erfinder:
- **Wolf, Heiko, Dr.
55288 Nierstein (DE)**
- **Dorn, Klaus, Dr.
63457 Hanau (DE)**
- **Eurich, Thomas
60388 Frankfurt (DE)**

(74) Vertreter: **Kahlhöfer, Hermann, Dipl.-Phys.
Patentanwälte
Kahlhöfer Neumann
Herzog Fiesser
Postfach 10 33 63
40024 Düsseldorf (DE)**

(56) Entgegenhaltungen:
**EP-A- 0 714 914**
**DE-A- 1 941 887**
**GB-A- 688 291**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 855 405 B1

Printed by Jouve, 75001 PARIS (FR)

## Beschreibung

Die Erfindung betrifft ein Absorptionsmaterial, das überwiegend auf nachwachsenden Rohstoffen basiert, das gegenüber den derzeit als Absorbermaterial überwiegend verwendeten Polyacrylaten eine verbesserte biologische Abbaubarkeit aufweist und das gegenüber zum gleichen Zweck vorgeschlagenen Stärkemaleaten eine verbesserte Alterungsbeständigkeit besitzt.

Insbesondere betrifft die Erfindung ein Verfahren zur Herstellung von quellbaren, alterungsbeständigen Stärkematerialien, die eine vergleichsweise hohe Aufnahmekapazität aufweisen und die im Vergleich zu anderen Absorbermaterialien auf Polysaccharidbasis nur eine sehr geringe Neigung zum Gelblocking zeigen. Alterungsbeständig bezieht sich in diesem Zusammenhang auf die Quellungseigenschaften und insbesondere nicht auf die biologische Abbaubarkeit. Die Erfindung offenbart des weiteren nach dem erfindungsgemäßen Verfahren erhältliches Absorptionsmaterial sowie die Verwendung der Produkte.

Die weitaus meisten der heute verwendeten Absorptionsmaterialien, häufig auch als Superabsorber bezeichnet, bestehen aus schwach vernetzten Polyacrylaten und sind somit nur zu einem sehr geringen Teil oder überhaupt nicht abbaubar (s. z. B. Stegman et al., Waste Manage. Res. 11 (1993) 155).

Neben den reinen Polyacrylaten gibt es auch auf Stärke gepfropfte Polyacrylate (DE-A 26 12 846). Der Stärkegehalt dieser Produkte ist mit bis zu 25 % jedoch gering. Bei höheren Stärkegehalten wird eine deutliche Verschlechterung der Absorptionseigenschaften beobachtet. Aufgrund des Polyacrylatgehaltes ist die biologische Abbaubarkeit auch dieser Produkte gering.

Ebenso können bis zu ca. 25 % eines zumindest begrenzt wasserlöslichen Polysaccharides in einen vernetzten Polyacrylatsuperabsorber eingearbeitet werden, indem das Polysaccharid während der Polymerisation des Acrylates in das Reaktionsgemisch eingebracht wird (DE-A 40 29 591, DE-A 40 29 592, DE-A 40 29 593).

US-A 5,079,354 beschreibt ein Absorbermaterial auf Basis von Carboxymethylstärke, also eines Stärkeethers, das durch Umsetzung von Stärke mit Chloressigsäure hergestellt wird. Bei diesem Prozeß wird eine äquivalente Menge Natriumchlorid bezogen auf die eingesetzte Chloressigsäure freigesetzt, was aus ökologischen Gründen unerwünscht ist. Ferner ist bekannt, daß veretherte Polysaccharide bei hohen Substitutionsgraden nur schlecht biologisch abbaubar sind (Mehltretter et al. J. Am. Oil Chem. Soc. 47 (1970) 522).

Die EP-A 0 603 837 beschreibt die Herstellung von Stärkeestern unter Verwendung von organischen Säureanhydriden. Hierzu läßt man Stärke diverser Herkunft in einem einstufigen, wässrigen Verfahren mit organischen Säureanhydriden der allgemeinen Formel I reagieren,

$$R - \overset{\overset{\displaystyle O}{\|}}{C} - O - \overset{\overset{\displaystyle O}{\|}}{C} - R \qquad \text{I}$$

worin R alkyl, aryl, alkenyl, alkaryl oder aralkyl mit 1 bis 7 C-Atomen bedeutet, unter bestimmten pH-, Temperaturund Konzentrationsbedingungen. Zu beispielhaft in der Beschreibung der EP-A 0 603 837 aufgezählten Stärkeestern gehören Stärkeacetat, -propionat, -butyrat, -hexanoat, - benzoat oder auch gemischte Stärkeacetate/propionate. In den Beispielen der EP-A 0 603 837 wird die Verwendung von Propionsäureanhydrid, Acetanhydrid und/oder Buttersäureanhydrid offenbart. Mit dem in der

EP-A 0 603 837 beschriebenen Verfahren gelingt es, die Probleme zu umgehen, die sich sonst beim Einsatz größerer Mengen Anhydrid ergeben haben, wie etwa das Quellen oder Gelantinieren der Stärke und Probleme beim Abtrennen der Stärkeester aus der Reaktionsmischung. Das Produkt wird nämlich durch die Umsetzung hydrophob und daher in einfacher Weise abfiltrierbar.

Aus der GB-A-688,291 sind Sulfonsäuregruppen enthaltende granuläre Stärken bekannt, die durch Modifizierung mit Sulfonsäuregruppen enthaltenden Reagenzien oder durch Umsetzung der Doppelbindungen von Maleatstärken mit Sulfonsäure bzw. deren Salzen gebildet werden. Die bei diesen Stärken verbesserten Eigenschaften, wie etwa Wasserabsorption, Verkleisterungstemperatur und geringere Neigung zum Gelieren von daraus hergestellten Pasten, werden ursächlich zurückgeführt auf die erhöhte Affinität der Sulfonsäuregruppen zum Wassermolekül im Vergleich zu Carboxylgruppen. Verwendet werden diese Stärken in Klebstoffen, als Verdicker in Lebensmitteln und bei der Herstellung von Textilien und Papier. Es wird weder die Verwendung der Stärken als Absorptionsmaterial in Hygieneprodukten, Verpackungsmaterial, Kabelummantelungen und zur Bodenverbesserung beschrieben, noch wird auf die Problematik der Alterungserscheinungen eingegangen.

Aus der WO 93/01217 (PCT/EP 92/01553) ist ein Verfahren zur Herstellung von Stärkeestern für klinische, insbesondere parenterale Anwendung bekannt. Die Stärkeester gemäß der WO 93/01217 sind sehr gut wasserlöslich,

was für die parenterale Anwendung zwingend erforderlich ist.

Es hat auch bereits Versuche gegeben, biologisch abbaubare Superabsorber zu schaffen. So kennt man aus der DE-A 42 06 857 ein Absorptionsmittel, das aus einer auf speziellen nachwachsenden Polysaccharid-Rohstoffen basierenden Komponente, einem speziellen wasserquellbaren Polymer, einem Matrixmaterial, einem ionischen oder kovalenten Vernetzer und einem Reaktivzusatz besteht. Die auf nachwachsenden Polysaccharid-Rohstoffen basierende Komponente umfaßt z. B. Guar, Carboxymethylguar, Xanthan, Alginate, Gummi Arabicum, Hydroxyethyl- oder Hydroxypropylcellulose, Carboxymethylcellulose und andere Cellulosederivate, Stärke und Stärkederivate wie Carboxymethylstärke. Ferner ist es aus der DE-A 42 06 857 bekannt, daß die aufgeführten Polymeren durch eine Vernetzung modifiziert werden können, um ihre Wasserlöslichkeit zu reduzieren und bessere Quelleigenschaften zu erreichen. Die Vernetzung kann sowohl im gesamten Polymeren stattfinden oder aber auch nur an der Oberfläche der einzelnen Polymerpartikel.

Die Umsetzung der Polymeren kann mit ionischen Vernetzern wie z. B. Calcium-, Aluminium-, Zirkon und Eisen(III)-Verbindungen erfolgen. Ebenso ist die Umsetzung mit polyfunktionellen Carbonsäuren wie Citronensäure, Schleimsäure, Weinsäure, Äpfelsäure, Malonsäure, Bernsteinsäure, Glutarsäure, Adipinsäure, mit Alkoholen wie Polyethylenglykole, Glycerin, Pentaerythrit, Propandiole, Saccharose, mit Kohlensäureestern wie Glykoldiglycidylether, Glykoldi- oder -triglycidylether und Epichlorhydrin, mit Säureanhydriden wie Bernsteinsäureanhydrid und Maleinsäureanhydrid, mit Aldehyden und mehrfunktionellen (aktivierten) Olefinen wie Bis-(acrylamido)-essigsäure und Methylenbisacrylamid möglich. Ebenso kommen natürlich Derivate der genannten Verbindungsklassen in Betracht sowie heterofunktionelle Verbindungen mit verschieden funktionellen Gruppen der oben genannten Verbindungsklassen.

Obwohl die vorgestellten und anhand von Beispielen belegten Systeme auf Basis von Natriumcarboxymethylcellulose in Verbindung mit Natriumpolyacrylat in verschiedenen Tests recht günstige Absorptionseigenschaften aufweisen, ist der Druckschrift in Bezug auf die biologische Abbaubarkeit nichts Näheres entnehmbar.

Es ist jedoch bekannt, daß Polyacrylate praktisch gar nicht (R. Stegmann et al. Waste Water Res. 11(2) (1993) S. 155) und Carboxymethylcellulose, ein Polysaccharidether nur sehr schlecht biologisch abbaubar ist (4,6 % nach 5 Tagen; M. Seekamp, Textilveredlung 25 (1990) S. 125).

Ein Absorptionsmaterial mit einer den derzeit überwiegend verwendeten Polyarcylaten gegenüber wesentlich verbesserten biologischen Abbaubarkeit wird in der EP-A-0 714 914 angegeben. Offenbart wird dort ein quellbarer Stärkeester, der zu mehr als 50 Gew.-% aus in Wasser unlöslichen Anteilen besteht und ein Rückhaltevermögen für 0,9 gew.-%ige wäßrige NaCl-Lösung von > 500 % aufweist, jeweils bezogen auf das Gewicht des trockenen Stärkeesters, wobei das Rückhaltevermögen dadurch bestimmt wird, daß man 0,1 g des in einen Nylonbeutel mit 52 µm Maschenweite eingeschweißten Stärkeesters für 30 min in einer 0,9 %igen NaCl-Lösung quellen läßt, den Beutel anschließend 5 min bei 1400 rpm zentrifugiert und danach eine ggf. resultierende Gewichtszunahme gravimetrisch bestimmt.

Obwohl die in der EP-A 0 714 914 vorgestellten, im wesentlichen auf Maleinsäureanhydrid und Stärke basierenden Absorptionsmaterialien sehr gute Quelleigenschaften und eine gute biologische Abbaubarkeit besitzen, sind die Stärkemaleate der EP-A 0 714 914 leider noch mit deutlichen Nachteilen behaftet.

So stellt man nach einigen Wochen eine deutliche Abnahme der Quelleigenschaften fest. Nach mehreren Wochen werden dann sowohl bei der Messung des Retentionsvermögens (SRV) als auch bei der Bestimmung des Absorptionsvermögens mit und ohne Last (AFK5 bzw. A20FK5) sehr schlechte Quelleigenschaften erhalten. Diese Alterung hat bislang die kommerzielle Anwendung von Stärkemaleaten als Absorptionsmaterialien stark behindert.

In Anbetracht des dargelegten und hierin diskutierten Standes der Technik ist es mithin eine Aufgabe der Erfindung gewesen, ein einfach durchführbares Verfahren zur Herstellung von quellbaren und alterungsbeständigen Stärkemaleaten anzugeben. Ferner sollen danach erhältliche biologisch abbaubare Stärkemaleate mit bezüglich der Quellungseigenschaften verminderter Alterung bereitgestellt werden. Noch eine Aufgabe der Erfindung besteht in der Verwendung von quellbaren, biologisch abbaubaren und hinsichtlich der Quellbarkeit alterungsbeständigen Stärkemaleaten.

Im Rahmen der Erfindung wurde in nicht ohne weiteres vorhersehbarer Weise gefunden, daß man quellbare und hinsichtlich der Quellungseigenschaften im wesentlichen alterungsbeständige Stärkemaleate herstellen kann, indem man ein quellbares Stärkemaleat mit einem oder mehreren ein- und/oder mehrfachfunktionellen SH-Gruppenhaltigen Nucleophil(en) nach Art einer Michael-Addition umsetzt.

Insbesondere ist auf diese Weise erhältliches, gegen Alterung der Quelleigenschaften beständige Material für den Einsatz als Absorptionsmaterial zu Absorption von Wasser, wäßrigen Lösungen, Dispersionen und Körperflüssigkeiten in Hygiene- und Tierhygiene, insbesondere in Windeln und Inkontinenzprodukten, sowie in Verpackungsmaterialien für Fleisch und Fisch, sowie zur Bodenverbesserung, zum Einsatz in Kulturgefäßen und als Kabelummantelungen geeignet, wobei es gleichzeitig immer noch biologisch abbaubar ist.

Die gemäß dem Verfahren der Erfindung umzusetzenden Stärkemaleate sind Stärkeester mit einem Substitutionsgrad zwischen 0,2 und 2,0, wobei der Substitutionsgrad die Anzahl der Substituenten pro Glucosering angibt. Bei den Estern kann es sich um reine Maleate handeln oder um gemischte Ester, d. h. neben Estergruppen, die sich

von der Maleinsäure ableiten, sind noch bis zu 98 %, vorzugsweise jedoch nur bis zu 50 %, besonders bevorzugt weniger als 50 %, z. B. 25-49 %, andere Estergruppen vorhanden. Trotzdem wird auch bei letzteren Verbindungen, selbst im ersten Fall, bei dem bis zu 98 % andere Estergruppen vorhanden sind, im Rahmen der Erfindung von Stärkemaleaten gesprochen. Grundsätzlich sind jedoch reine Maleate für die Erfindung ebenfalls bestens geeignet.

Die Stärkemaleate werden bevorzugt durch Umsetzung von Stärke mit Maleinsäureanhydrid und ggf. weiteren Säureanhydriden erhalten, wobei es sich bei den weiteren Anhydriden um cyclische und/oder offenkettige Anhydride handeln kann. Erfindungsgemäß ist jedoch die alleinige Umsetzung mit Maleinsäureanhydrid bei weitem bevorzugt.

Zu den Carbonsäureanhydriden, die besonders vorteilhaft zusammen mit Maleinsäureanhydrid eingesetzt werden, gehören u. a. Acetanhydrid, Propionsäureanhydrid und/oder Bernsteinsäureanhydrid.

Obwohl die Verbesserung der Alterungsbeständigkeit gemäß der Erfindung bei jedem quellbaren Stärkemaleat erzielt werden kann, werden in einer ersten bevorzugten Verfahrensvariante quellbare Stärkemaleate umgesetzt, die nach einem Verfahren erhältlich sind, bei dem Stärke oder modifizierte Stärke in einer einstufigen wässrigen Reaktion mit Maleinsäureanhydrid oder einer Mischung von Carbonsäureanhydriden aufweisend Maleinsäureanhydrid bei einem pH-Wert von 7 bis 11 und einer Temperatur von 0 bis 40 °C umgesetzt wird, wobei der pH-Wert durch Zugabe von wässriger Alkali-Lösung mit einer Konzentration von ca. 10 bis 50 Gew.-% im gewünschten Bereich gehalten wird.

Während der Reaktion des Anhydrides oder der Anhydride mit der Stärke wird der pH vorzugsweise konstant gehalten. Der pH sollte während der Reaktion zwischen 7 und 11 liegen. Bevorzugt wird ein pH Wert zwischen 8 und 9. Der pH kann im Prinzip durch Zugabe eines beliebigen alkalischen Materials konstant gehalten werden. Besonders nützlich sind Alkaliund Erdalkalihydoxide sowie die Oxide und Carbonate der Alkali- und/oder Erdalkalimetalle. Beispielhaft genannt seien Natriumhydroxid, Kaliumhydroxid, Calciumhydroxid, Ammoniumhydroxid, Magnesiumhydroxid und Natriumcarbonat. Bei der Herstellung von Produkten mit einem höheren Substitutionsgrad wird bevorzugt eine Alkalilösung mit einer höheren Konzentration, ca. 10 bis 50 %ig, verwendet um eine unnötige Verdünnung des Reaktionsmediums zu vermeiden.

Eine zweckmäßige zweite Abwandlung des erfindungsgemäßen Verfahrens sieht vor, daß ein quellbares Stärkemaleat eingesetzt wird, welches nach einem Verfahren erhältlich ist, bei dem Stärke oder modifizierte Stärke mit Maleinsäureanhydrid oder einer Mischung von Carbonsäureanhydriden aufweisend Maleinsäureanhydrid in Gegenwart einer Base und ggf. eines Lösungsmittels umgesetzt wird, wobei die Abwandlung sich dadurch kennzeichnet, daß man bei einer Temperatur von 80 bis 180 °C für 10 min bis 10 h reagieren läßt, und daß Lösungsmittel nur bis zu einem Gehalt von weniger als 100 Teile bezogen auf 100 Teile Stärke toleriert wird.

Bevorzugt ist für diese Verfahrensmodifikation, daß als Base Natriumcarbonat verwendet wird.

Als Stärkebasis der erfindungsgemäß einsetzbaren Stärkemaleate kann dabei im Prinzip jede native, modifizierte oder substituierte Stärke verwendet werden. Derartige Stärken können aus einer beliebigen pflanzlichen Quelle isoliert worden sein, und umfassen z. B. Kartoffelstärke, Maisstärke, Weizenstärke, Wachsmaisstärke und Stärken mit hohem Amylosegehalt. Stärkemehl kann ebenfalls verwendet werden. Auch verwendet werden können modifizierte Produkte auf Basis einer der oben aufgeführten Stärken wie z. B. säurehydrolysierte Stärke, enzymhydrolysierte Stärke, Dextrine und oxidierte Stärke. Des weiteren können derivatisierte Stärken wie kationische Stärke, anionische Stärke, amphotere Stärke oder nichtionisch modifizierte Stärke wie z. B. Hydroxyethylstärke verwendet werden. Bei den verwendeten Stärken kann es sich um granuläre oder vorgelatinisierte Stärke handeln, wobei die Zerstörung der granulären Struktur thermisch, mechanisch oder chemisch erfolgen kann.

Besonders vorteilhaft für die Erfindung ist der Einsatz von Stärkemaleaten auf Basis kaltwasserlöslicher Stärke. Hierunter versteht man insbesondere vorgelatinierte oder partiell abgebaute Stärke. Hierzu gehört u. a. Aeromyl 115 der Firma Südstärke.

Gemäß der Erfindung wird ein quellbares Stärkemaleat mit einem oder mehreren Nucleophil(en) nach Art einer Michael-Addition zur Reaktion gebracht. Hierbei kommt es vermutlich zur Anlagerung des Nucleophils an die Doppelbindung der Maleinsäure im Stärkemaleat.

Zu den erfindungsgemäß hierzu verwendbaren Nucleophilen gehören dabei vor allem solche der allgemeinen Formel I

$$H—X—R \qquad (I),$$

worin

X für S und

R für Wasserstoff, einen gesättigten oder ungesättigten Alkylrest mit 1 bis 18 Kohlenstoffatomen steht, wobei der Alkylrest verzweigt oder unverzweigt ist und optional innerhalb der Kette ein oder mehrere Sauerstoffatome aufweisen kann und weiters optional mit anderen funktionellen Gruppen substituiert sein kann, wie beispielsweise Carboxyl, Hydroxyl, Carbonyl, Ester, Amid, Ether, Sulfid, Halogenid, Sulfonsäure, oder für einen Arylrest mit 6-12

C-Atomen steht, wobei der Arylrest bis zu vierfach mit Carboxyl, Hydroxyl, Carbonyl, Ester, Amid, Sulfonsäure und/oder Halogenid substituiert sein kann.

Besonders günstig einsetzbar sind auch Salze der Verbindungen der Formel I, worin wenigstens ein Wasserstoff durch Alkali oder Erdalkali ersetzt sein kann. Entsprechende Verbindung ist z. B. $Na_2S$.

Weiterhin erfindungsgemäß besonders günstig verwendbar sind Nucleophile der allgemeinen Formel II

$$H—X—R—Y—H \qquad (II),$$

worin

X und R die bei Formel I angegebene Bedeutung besitzen und

Y unabhängig von X gleich oder verschieden hierzu S oder NH bedeutet. Auch von Verbindungen der Formel II sind wie bei Formel I-Verbindungen Salze einsetzbar.

Sofern von den erfindungsgemäß einsetzbaren Verbindungen der Formeln I oder II optische aktive Formen existieren, gehören diese sowohl einzeln als auch im Gemisch, beispielsweise als Enantiomere, Diastereomere, Racemate, zur Erfindung.

Sowohl die Nucleophile der allgemeinen Formel I als auch der allgemeinen Formel II lagern sich im Sinne einer Michael-analogen Addition an die Doppelbindung der in den Stärkemaleaten vorhandenen Maleinsäure-Bausteine an. Bei den Thiolen ist im allgemeinen keine zusätzliche Katalyse nötig, um eine schnelle Addition an die Doppelbindungen zu ermöglichen.

Es ist bemerkenswert, daß der erfindungsgemäß angestrebte Effekt der Verhinderung des Alterns von Stärkemaleaten sowohl bei den monofunktionellen Bausteinen der allgemeinen Formel I durch Blockade der in Frage stehenden Doppelbindung der Maleatgruppe als auch durch die difunktionellen Bausteine der allgemeinen Formel II durch Vernetzung und somit ebenfalls Blockade der Doppelbindung der Maleatgruppe eintritt.

Zu einzelnen im Rahmen des erfindungsgemäßen Verfahrens einsetzbaren monofunktionellen Bausteinen gehören u. a. lineare und verzweigte **Thiole**, wie Ethanthiol, 1-Propanthiol, 2-Propanthiol, 1-Butanthiol, sec.-Butylmercaptan, tert.-Butylmercaptan, Isobutylmercaptan, 1-Pentanthiol, 1-Hexanthiol, 1-Heptanthiol, 1-Octanthiol, 1-Nonanthiol, 1-Decanthiol, 1-Undecanthiol, 1-Dodecanthiol, tert.-Dodecanthiol, 1-Tetradecanthiol, 1-Hexadecanthiol, 1-Octadecanthiol, Mercaptoethanol, 3-Mercapto-1-propanol, 4-Mercapto-1-butanol, 3-Mercapto-2-butanol, 6-Mercapto-1-hexanol, 8-Mercapto-1-octanol, 10-Mercapto-1-decanol, 11-Mercapto-1-undecanol, Mercaptoessigsäure und deren Salze und Ester, 3-Mercaptopropionsäure und deren Salze und Ester, 11-Mercaptoundecansäure und deren Salze und Ester, Mercaptoethansulfonsäure und deren Salze, 3-Mercapto-1-propansulfonsäure und deren Salze, Mercaptobrenztraubensäure und deren Salze, 2-Diethylaminoethanthiol(-hydrochlorid), 3-Mercaptopropylmethyldimethoxysilan, 3-Mercaptopropyl-triethoxysilan, 3-Mercaptopropyl-trimethoxysilan, N-Methylmercaptoacetamid, Mercaptophenol.

Der Einsatz von Mercaptoethanol, 3-Mercapto-1-propanol, Mercaptoessigsäure, 3-Mercaptopropionsäure, Mercaptoethansulfonsäure und Mercaptopropansulfonsäure ist dabei besonders bevorzugt.

Zu den im Rahmen der Erfindung einsetzbaren difunktionellen Vernetzermolekülen gehören u. a. **Aminosäuren mit zusätzlichen Thiolgruppen** wie Cystein, Penicillamin,

**Dithiole** wie Ethandithiol, 1,3-Propandithiol, 1,4-Butandithiol, 2,3-Butandithiol, 1,5-Pentandithiol, 1,6-Hexandithiol, 1,7-Hepatandithiol, 1,8-Octandithiol, 1,9-Nonandithiol, 1,10-Decandithiol, 1,11-Undecandithiol, 1,12-Dodecandithiol, 1,16-Hexadecandithiol;

**Aminothiole** wie Cysteamin, 2-Mercapto-isobutylamin;

Bis-(2-mercaptoethyl)-ether, Bis-(2-mercaptoethyl)-sulfid, 1,2-Bis-(2-mercaptoethoxy)-ethan (MEE), N-(2-Mercaptopropionyl)-glycin, 2,3-Dimercapto-bernsteinsäure, 2,3-Dihydroxy-1,4-butandithiol, 2,3-Dimercapto-1-propanol, 2,3-Dimercapto-propansulfonsäure und deren Salze, Liponsäure (reduzierte Form), Pentaerythrit-tetrakis-(3-mercapto-propionat), Pentaerythrit-tetrakis-(2-mercaptoacetat), gemischte Ester der 2-Mercaptoessigsäure und der 3-Mercaptoessigsäure mit Pentaerythrit, Schwefelwasserstoff, Natriumsulfid, 1,3-Dimercapto-benzol, 1,2-Dimercapto-benzol, Toluol-3,4-dithiol, Oligosulfide mit terminalen Thiolgruppen (Thiokol-Produkte der Firma Morton), weiterhin Oligo- und Polypeptide mit mindestens zwei freien Amino- und/oder Thiolgruppen.

Für den Einsatz als Nucleophil im Rahmen des erfindungsgemäßen Verfahrens ganz besonders bevorzugt sind 1,2-Bis-(2-mercaptoethoxy)-ethan) (MEE), 2,3-Dimercapto-bernsteinsäure, Cystein, Cysteamin, 2,3-Dihydroxy-1,4-butandithiol, Pentaerythrit-tetrakis-(3-mercaptopropionat), Pentaerythrit-tetrakis-(2-mercapto-acetat), Bis-(2-mercaptoethyl)-ether, Bis-(2-mercaptoethyl)-sulfid.

Ein ganz besonders günstiger Effekt läßt sich im Rahmen der Erfindung dadurch erzielen, daß man einen difunktionellen Vernetzer (Nucleophil der Formel II) und einen monofunktionellen Baustein (Nucleophil der Formel I)

kombiniert. Gerade für die kombinierte Verwendung eines monofunktionellen mit einem difunktionellen Baustein werden beste Quelleigenschaften mit einer weitestgehenden Verhinderung der Alterung von Stärkemaleaten realisiert. Somit kennzeichnet sich das Verfahren der Erfindung in einer weiteren ganz besonders zweckmäßigen Abwandlung dadurch, daß man eine Mischung aus Mercaptoethanol (ME) und 1,2 Bis-(2-mercaptoethoxy)-ethan (MEE), oder eine Mischung aus Pentaerythrit-tetrakis-(2-mercapto-acetat) und Natriumbisulfit einsetzt. Durch die Vernetzung von Stärkemaleaten mit Di- oder Oligothiolen und die Umsetzung von ggf. restlichen Maleat-Doppelbindungen mit Thiolen oder Natriumbisulfit lassen sich insbesondere in einfacher Weise Netzwerkaufbau und Alterungsstabilität miteinander kombinieren.

Daher ist es für das Verfahren der Erfindung weiterhin vorteilig, daß man zwischen 0,001 und 0,5 eq Mercaptoethoxyethan (MEE) in Kombination mit zwischen 0,5 und 0,999 eq Mercaptoethanol (ME) als Nucleophil einsetzt, wobei die Äquivalente sich auf die im Stärkemaleat enthaltenen Maleinsäureanhydrid-Mengen beziehen. Innerhalb dieser Vernetzer- und Blockierungsmittel-Konzentration können so hinsichtlich des SRV und der freien Quellung gute Quellwerte eingestellt werden. Vorzügliche Bereiche liegen zwischen 0,001 und 0,03 eq MEE und 0,97 und 0,999 ME.

Weiterhin vorteilig ist es für das Verfahren, daß man zwischen 0,001 und 0,5 eq Pentaerythrit-tetrakis-(2-mercapto-acetat) (PTMA) in Kombination mit zwischen 0,25 und 0,999 eq Natriumbisulfit als Nucleophil einsetzt, wobei die Äquivalente sich auf die im Stärkemaleat enthaltenen Maleinsäureanhydrid-Mengen beziehen. Innerhalb dieser Vernetzer- und Blockierungsmittel-Konzentration können so hinsichtlich des SRV und der freien Quellung gute Quellwerte eingestellt werden. Vorzügliche Bereiche liegen zwischen 0,001 und 0,03 eq PTMA und 0,4 und 0,9 Natriumbisulfit.

Obwohl die Umsetzung von Stärkemaleaten mit Nucleophilen im Rahmen der Erfindung mit Stärkemaleaten jeglicher Herkunft durchführbar ist, spielt das Verfahren der Erfindung seine besonderen Vorteile jedoch insbesondere dann aus, wenn die Stärkemaleate im gequollenen oder gelösten Zustand mit einem Nucleophil behandelt werden. Insbesondere im Anschluß an die eigentliche Synthesereaktion der Stärkemaleate kann dadurch die Umsetzung in einer Eintopfreaktion durchgeführt werden. Insofern ist es eine bevorzugte Abwandlung des erfindungsgemäßen Verfahrens, daß man die Umsetzung mit dem einen und/oder mehreren ein- und/oder mehrfach funktionellen Nucleophil (en) unmittelbar anschließend an die Synthese des Stärkemaleats in einer Eintopfreaktion bei Temperaturen zwischen Raumtemperatur und etwa 80 °C über einen Zeitraum von 10 min bis 24 h, vorzugsweise zwischen 10 min und 10 h, durchführt.

Insbesondere durch diese extrem einfache Versuchsdurchführung lassen sich im Rahmen der Erfindung durch die Vernetzung der Stärkemaleate bevorzugt mit Dithiolen und die Umsetzung der restlichen Maleat-Doppelbindungen mit bevorzugt Thiolen Netzwerkaufbau und Alterungsstabilität optimal miteinander kombinieren und kontrollieren.

Gegenstand der Erfindung sind auch nach dem hierin beschriebenen Verfahren erhältliche biologisch abbaubare Stärkemaleate. Diese kennzeichnen sich durch das überaus günstige Alterungsverhalten bei gleichzeitig guten Quelleigenschaften. Insbesondere kennzeichnen sich die biologisch abbaubaren Stärkemaleate gemäß der Erfindung dadurch, daß eine Abnahme des SRV nach 100 Tagen von weniger als 20 % feststellbar ist. Weiters sind besonders bevorzugte erfindungsgemäße Stärkemaleate solche, welche eine Abnahme des SRV nach 100 Tagen von weniger als 10 % aufweisen.

Eine besonders zweckmäßige Verfahrensweise sieht vor, das Gelblocking der hergestellten Superabsorber Partikel zu verringern. Wie allgemein von Superabsorber Partikeln bekannt, zeigen schwach vernetzte Produkte zwar eine höhere Flüssigkeitsaufnahme als stark vernetzte, sie neigen aber auch in größerem Maße zu Gelblocking, speziell bei Absorption unter Last. Um das Gelblocking schwach vernetzter Gele zu verhindern, wird erfindungsgemäß ein Stärkemaleat mit einer Verbindung der Formel I und/oder Formel II oder einer Mischung in der Art umgesetzt, daß ein schwach vernetztes Gel entsteht, dessen Maleatdoppelbindungen nur zum Teil abreagiert sind. Nach dem Trocknen und Zerkleinern wird das Produkt in einem Lösungsmittel oder Lösungsmittelgemisch suspendiert, in dem ein Vernetzer der Formel II oder eine Mischung aus Verbindungen nach Formel II und Formel I gelöst ist. Nach diesem Verfahren findet eine zusätzliche Vernetzung in erster Linie an der Oberfläche der Partikel statt. Die Superabsorbergele zeigen nach dieser Behandlung eine deutlich geringere Neigung zu Gelblocking, behalten aber ein wesentlich besseres Absorptionsvermögen als durchgehend hochvernetzte Gele.

Als Lösungsmittel für die erfindungsgemäße Nachvernetzung eignen sich organische Lösungsmittel, die mit Wasser mischbar sind. Bevorzugt sind Alkohole wie Methanol, Ethanol, Propanol und i-Propanol und deren Mischungen mit Wasser im Verhältnis 50:50 bis 99:1. Besonders bevorzugt sind Mischungen von Ethanol mit Wasser im Verhältnis 70:30 bis 99:1.

Gegenstand der Erfindung ist auch die Verwendung der genannten Stärkemaleate in einer Menge von 100 Gew.-Teilen zusammen mit 0,7 bis 70 Gew.-Teilen eines Antiblocking-Mittels auf Basis natürlicher oder synthetischer, bevorzugt hydrophiler Fasern oder Materialien mit großer Oberfläche als Superabsorber. Bevorzugt ist die Verwendung des Stärkemaleats als Superabsorber zusammen mit 1 bis 5 Gew.-Teilen Kieselsäure oder Cellulosefasern als Antiblocking-Mittel. Weitere Anwendung findet der Stärkeester der Erfindung als Absorptionsmittel zur Absorption von

Wasser, wäßrigen Lösungen, Dispersionen und Körperflüssigkeiten in Hygiene und Tierhygiene, insbesondere in Windeln, Tampons und Inkontinenzprodukten, sowie in Verpackungsmaterialien für Fleisch und Fisch, als Absorptionsmaterial zur Absorption von Wasser und wäßrigen Lösungen in Kulturgefäßen und zur Bodenverbesserung oder als Absorptionsmaterial zur Absorption von Wasser und wäßrigen Lösungen in Kabelummantelungen.

Um bei erfindungsgemäß alterungsbeständigen Stärkemaleaten das Verhalten unter Druckbelastung weiter zu verbessern, ist es ferner besonders vorteilig, die Partikeloberfläche der Stärkemaleate nachzuvernetzen. Hierdurch kann ein Gelblocking unter Beibehaltung guter Werte im SRV und AFK5 verhindert werden.

Nachfolgend wird die Erfindung anhand von Ausführungsbeispielen näher erläutert.

## I) Methoden

### A) Bestimmung der Substitutionsausbeute über den NaOH-Verbrauch während der Synthese

Bei der Umsetzung von Stärke mit Maleinsäureanhydrid (MSA)in wäßriger Lösung wird pro mol Maleinsäureanhydrid ein mol NaOH verbraucht. Findet keine Addition des Maleinsäureanhydrides an die Stärke sondern eine Hydrolyse zum Dinatriummaleat statt, so werden, bei pH-Konstanthaltung, pro Mol MSA 2 Mol NaOH verbraucht. Wenn am Ende der Reaktion kein nicht reagiertes Anhydrid mehr vorliegt, läßt sich der Substitutionsgrad nach

$$DS(NaOH) = DS_{theoret}\left(1 - \frac{Verbrauch\ an\ NaOH\ in\ Mol - eingesetzte\ Menge\ MSA\ in\ Mol}{eingesetzte\ Menge\ MSA\ in\ Mol}\right)$$

berechnen, wobei

$$DS_{theoret} = \frac{eingesetzte\ Menge\ MSA\ in\ Mol}{eingesetzte\ Menge\ Stärke\ in\ Mol_{Monosaccharid}}$$

bedeutet.

### B) Methode zur Bestimmung des Rückhaltevermögens (Retentionsvermögens) = SRV,

Zur Bestimmung des Retentionsvermögens wurde ein Teebeutel-Test durchgeführt. Hierzu werden 0,10 g der Prüfsubstanz in einen Beutel aus Nylongewebe mit einer Maschenweite von 52 µm eingewogen. Der verschweißte Nylonbeutel wird in eine 0,9 %ige NaCl-Lösung gegeben und das Prüfmaterial wird 30 Minuten quellen gelassen. Anschließend wird der Beutel herausgenommen und 5 Minuten bei 1400 Upm in einem Zentrifugeneinsatz mit Siebboden geschleudert. Die Flüssigkeitsaufnahme wird gravimetrisch bestimmt und auf 1 g der zu prüfenden Substanz umgerechnet. Der so erhaltene Wert wird als Rückhaltevermögen (Retentionsvermögen) bezeichnet (abgekürzt SRV für saline retention value).

### C) Methode zur Bestimmung des Absorptionsvermögens mit und ohne Last (AFK5, A20FK5)

Zu 95 Teilen der zu untersuchenden Substanz werden 5 Teile Fällungskieselsäure (FK500 LS, Degussa) gemischt. Diese Mischung wird im weiteren als Testsubstanz bezeichnet.

0,5 g der Testsubstanz werden auf eine G3-Glasfritte mit 3 cm Durchmesser gegeben. Die Fritte ist mit einer Bürette über einen Schlauch verbunden, wobei die Bürette mit 0,9 %iger NaCl-Lösung gefüllt ist. Es wird die Flüssigkeitsmenge bestimmt, die die Testsubstanz in 10 min aufsaugt. Während der Versuchsdauer wird die Bürette so nachgeführt, daß der Meniskus immer auf Höhe der Glasfritte ist. Der so bestimmte Wert wird auf 1 g Testsubstanz umgerechnet und mit AFK5 bezeichnet.

Zur Bestimmung des Absorptionsvermögens unter Last (A20FK5) wird in der oben beschriebenen Ausführung zusätzlich ein Gewicht auf die Testsubstanz gestellt, das einen Druck von 20 g/cm$^2$ ausübt. Der so erhaltene Wert wird ebenfalls auf 1 g Testsubstanz umgerechnet.

## II) Beispiele und Vergleichsbeispiele

### Beispiel 1 - Synthese von Stärkemaleat

50 g (= 0,278 mol) Aeromyl 115 (physikalisch modifizierte, kaltwasserlösliche Stärke der Firma Südstärke; Restfeuchte = ca. 9,5 %) werden in 400 ml Wasser gelöst. Der pH wird mit 3 N NaOH auf 8 eingestellt und während der Reaktion konstant gehalten. Bei einer Reaktionstemperatur von 0 °C werden 27,2 g (= 0,278 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,88.

### Beispiel 2 - Synthese von Stärkemaleat

50 g (= 0,278 mol) Aeromyl 115 (physikalisch modifizierte, kaltwasserlösliche Stärke der Firma Südstärke; Restfeuchte = ca. 9,5 %) werden in 400 ml Wasser gelöst. Der pH wird mit 3 N NaOH auf 8 eingestellt und während der Reaktion konstant gehalten. Bei einer Reaktionstemperatur von 0 °C werden 40,91 g (= 0,417 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 1,15.

### Beispiele 3-8

Unmittelbar nach der einstündigen Nachreaktion des Stärkemaleats aus Beispiel 1 wird auf 60°C geheizt und eine Vernetzer-Mischung aus 1,2-Bis-(2-mercaptoethoxy-ethan) (MEE) und/oder Mercaptoethanol (ME) in den in Tabelle 2 angegebenen Mengen zum Reaktionsgemisch gegeben. Der pH-Wert bei der Zugabe liegt zwischen 7 und 8. Es wird homogenisiert und das Rührwerk abgeschaltet. Nach ca. 1 Minute tritt Gelbildung ein. Man läßt 3 h bei 60°C nachreagieren und kühlt das Reaktionsgemisch über Nacht ab. Am Rotavapor wird das Wasser bei 80°C im Vakuum abgezogen und über Nacht im Vakuumtrockenschrank bis zur Gewichtskonstanz getrocknet. Das so gewonnene Stärkemaleat wird zerkleinert und gemahlen. Von dem so gewonnenen Produkt wird das Absorptionsvermögen (AFK5; A20FK5) und das Retentionsvermögen (SRV) bestimmt. Die Messungen werden nach Lagerung wiederholt (siehe Tabelle 2). Tabelle 2 gibt die Veränderungen im SRV wieder, da hier die Alterungseffekte erfahrungsgemäß am deutlichsten auftreten (siehe auch die Vergleichsbeispiele).

Die Vernetzung mit MEE/ME läßt sich auch bei Raumtemperatur durchführen. Die Zeit bis zur Gelbildung verlängert sich dann auf 20 - 45 Minuten. Nachreaktion bei Raumtemperatur über Nacht und Aufarbeitung werden genauso durchgeführt.

Tabelle 2

| Übersicht der synthetisierten Produkte. Die Mengenangaben unter "Erläuterung" entsprechen mol-Äquivalenten bezüglich Maleinsäureanhydrid. Die Trocknung geschah bei 80 °C. | | | | |
|---|---|---|---|---|
| **Bsp.** | **Erläuterung** | **SRV [g/g] Anfangswert** | **SRV [g/g] nach (x) Tagen** | **Abweichung [%]** |
| 3 | Stärkemaleat,<br>0,5 eq MEE | 5,6 | 5,6 (128) | **0** |
| 4 | Stärkemaleat,<br>0,25 eq MEE<br>0,5 eq ME | 5,3 | 5,4 (128) | **+2** |
| 5 | Stärkemaleat,<br>0,1 eq MEE<br>0,8 eq ME | 7,9 | 7,5 (122) | **-5** |
| 6 | Stärkemaleat,<br>0,03 eq MEE<br>0,97 eq ME | 15,9 | 14,1 (127) | **-11** |

Tabelle 2 (fortgesetzt)

| Übersicht der synthetisierten Produkte. Die Mengenangaben unter "Erläuterung" entsprechen mol-Äquivalenten bezüglich Maleinsäureanhydrid. Die Trocknung geschah bei 80 °C. | | | | |
|---|---|---|---|---|
| **Bsp.** | **Erläuterung** | **SRV [g/g] Anfangswert** | **SRV [g/g] nach (x) Tagen** | **Abweichung [%]** |
| 7 | Stärkemaleat, 0,01 eq MEE 0,99 eq ME | 12,5 | 12,4 (115) | **-1** |
| 8 | Stärkemaleat, 0,005 eq MEE 0,98 eq ME | 13,3 | 12,9 (115) | **-3** |

**Beispiel 9 - Synthese eines gemischten Stärkeesters**

50 g (= 0,278 mol) Aeromyl 115 (physikalisch modifizierte, kaltwasserlösliche Stärke der Firma Südstärke; Restfeuchte = ca. 9,5 %) werden in 400 ml Wasser gelöst. Der pH wird mit 3 N NaOH auf 8 eingestellt und während der Reaktion konstant gehalten. Bei einer Reaktionstemperatur von 0 °C wird eine Mischung aus 1,088 g (0,011 mol) festem Maleinsäureanhydrid und aus 26,71 g (0,2668 mol) festem Bernsteinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,88.

Unmittelbar nach der einstündigen Nachreaktion des Stärkemaleats wird auf 60°C geheizt und 0,506g (0,00278 mol)des Vernetzers 1,2-Bis-(2-mercaptoethoxy-ethan) (MEE) zum Reaktionsgemisch gegeben. Der pH-Wert bei der Zugabe liegt zwischen 7 und 8. Es wird homogenisiert und das Rührwerk abgeschaltet. Nach ca. 25 Minuten tritt Gelbildung ein. Man läßt 3 h bei 60°C nachreagieren und kühlt das Reaktionsgemisch über Nacht ab. Am Rotavapor wird das Wasser bei 45°C im Vakuum abgezogen und über Nacht im Vakuumtrockenschrank bis zur Gewichtskonstanz getrocknet. Das so gewonnene Stärkemaleat wird zerkleinert und gemahlen.

SRV = 23,4 g/g

**Beispiel 10**

Das Produkt aus Beispiel 9 wird mit 5 % einer Fällungskieselsäure (FK500LS, Degussa AG) vermischt.
AFK5 = 10,2 ml/g; A20FK5 = 3,4 ml/g

**Beispiel 11**

100 g (= 0,568 mol) Aeromyl 115 (physikalisch modifizierte, kaltwasserlösliche Stärke der Firma Südstärke; Restfeuchte = ca. 7,9 %) werden in 800 ml Wasser gelöst. Der pH wird mit 3 N NaOH auf 8 eingestellt und während der Reaktion konstant gehalten. Bei einer Reaktionstemperatur von 0 °C werden 55,7 g (= 0,568 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,86. Anschließend werden 48,6 g Natriumbisulfit in 100 ml Wasser und 3,11 g 1,2-Bis-(2-mercaptoethoxy-ethan) (MEE) in 25 ml Ethanol zugegeben. Es wird homogenisiert und das Rührwerk abgeschaltet. Man läßt über Nacht nachreagieren. Am Rotavapor wird das Wasser bei 80°C im Vakuum abgezogen und das Produkt über Nacht im Vakuumtrockenschrank bis zur Gewichtskonstanz getrocknet. Das so gewonnene Stärkemaleat wird zerkleinert und gemahlen.
SRV = 17,4 g/g; AFK5 = 20,6 ml/g; A20FK5 = 3,9 ml/g
SRV nach 126 Tagen = 16,0 g/g (-8 %)

**Beispiel 12**

100 g (= 0,568 mol) Aeromyl 115 (physikalisch modifizierte, kaltwasserlösliche Stärke der Firma Südstärke; Restfeuchte = ca. 7,9 %) werden in 800 ml Wasser gelöst. Der pH wird mit 3 N NaOH auf 8 eingestellt und während der Reaktion konstant gehalten. Bei einer Reaktionstemperatur von 0 °C werden 27,8 g (= 0,284 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,48. Anschließend werden 24,3g Natriumbisulfit in 100 ml Wasser und 3,69 g Pentaerythrit-tetrakis-(2-mercapto-acetat) (PTMA) in 25 ml THF zugegeben. Es wird homogenisiert und das Rührwerk abgeschaltet. Man läßt über Nacht nachreagieren. Am Rotavapor wird das Wasser bei 80°C im Vakuum abgezogen und das Produkt über Nacht im Vakuumtrockenschrank bis zur Gewichtskonstanz getrocknet. Das so gewonnene Stärkemaleat wird zerkleinert und gemahlen.
SRV = 14,2 g/g; AFK5 = 21,3 ml/g; A20FK5 = 6,4 ml/g
SRV nach 106 Tagen = 12,6 g/g (-11 %)

**Beispiel 13**

100 g (= 0,568 mol) Aeromyl 115 (physikalisch modifizierte, kaltwasserlösliche Stärke der Firma Südstärke; Restfeuchte = ca. 7,9 %) werden in 800 ml Wasser gelöst. Der pH wird mit 3 N NaOH auf 8 eingestellt und während der Reaktion konstant gehalten. Bei einer Reaktionstemperatur von 0 °C werden 27,8 g (= 0,284 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,45. Anschließend werden 24,3g Natriumbisulfit in 100 ml Wasser zugegeben. Man erhitzt die Reaktionsmischung auf 40°C und gibt 1,84 g Pentaerythrit-tetrakis-(2-mercaptoacetat)(PTMA) in 25 ml THF zu. Es wird homogenisiert und das Rührwerk abgeschaltet. Man läßt 2h bei 40°C und über Nacht bei Raumtemperatur nachreagieren. Das Produkt wird im Umlufttrockenschrank bei 50°C getrocknet. Das so gewonnene Stärkemaleat wird zerkleinert und gemahlen.
SRV = 17,5 g/g; AFK5 = 19,2 ml/g; A20FK5 = 3,8 ml/g
SRV nach 118 Tagen = 15,7 g/g (-10 %)

**Beispiel 14**

100 g (= 0,568 mol) Aeromyl 115 (physikalisch modifizierte, kaltwasserlösliche Stärke der Firma Südstärke; Restfeuchte = ca. 7,9 %) werden in 800 ml Wasser gelöst. Der pH wird mit 3 N NaOH auf 8 eingestellt und während der Reaktion konstant gehalten. Bei einer Reaktionstemperatur von 0 °C werden 27,8 g (= 0,284 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,45. Anschließend werden 24,3g Natriumbisulfit in 100 ml Wasser zugegeben. Man erhitzt die Reaktionsmischung auf 60°C und gibt 1,84 g Pentaerythrit-tetrakis-(2-mercaptoacetat)(PTMA) in 25 ml THF zu. Es wird homogenisiert und das Rührwerk abgeschaltet. Man läßt 2h bei 60°C und über Nacht bei Raumtemperatur nachreagieren. Das Produkt wird im Umlufttrockenschrank bei 50°C getrocknet. Das so gewonnene Stärkemaleat wird zerkleinert und gemahlen.
SRV = 15,8 g/g; AFK5 = 20,8 ml/g; A20FK5 = 4,1 ml/g
SRV nach 112 Tagen = 14,6 g/g (-8 %)

**Beispiel 15**

100 g (= 0,568 mol) Aeromyl 115'(physikalisch modifizierte, kaltwasserlösliche Stärke der Firma Südstärke; Restfeuchte = ca. 7,9 %) werden in 800 ml Wasser gelöst. Der pH wird mit 3 N NaOH auf 8 eingestellt und während der Reaktion konstant gehalten. Bei einer Reaktionstemperatur von 0 °C werden 27,8 g (= 0,284 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,45. Anschließend werden 24,3g Natriumbisulfit in 100 ml Wasser und 0,92 g Pentaerythrit-tetrakis-(2-mercapto-acetat) (PTMA) in 25 ml THF zugegeben. Es wird homogenisiert und das Rührwerk abgeschaltet. Man läßt über Nacht nachreagieren. Am Rotavapor wird das Wasser bei 80°C im Vakuum abgezogen und das Produkt über Nacht im Vakuumtrockenschrank bis zur Gewichtskonstanz getrocknet. Das so gewonnene Stärkemaleat wird zerkleinert und gemahlen.
SRV = 20,6 g/g; AFK5 = 21,2 ml/g; A20FK5 = 4,2 ml/g
SRV nach 106 Tagen = 18,0 g/g (-13 %)

**Vergleichsbeispiel 1**

Die Reaktionslösung aus Beispiel 1 wird einrotiert und im Vakuumtrockenschrank nachgetrocknet. Das Produkt wird gemahlen und nochmals 30 min bei 100°C im Vakuumtrockenschrank nachgetrocknet.
SRV = 14,3 g/g;

**Vergleichsbeispiel 2**

Das Produkt aus Vergleichsbeispiel 1 wird mit 5 % einer Fällungskieselsäure (FK500LS, Degussa AG) vermischt.
AFK5 = 21,1 ml/g; A20FK5 = 7,6 ml/g

**Vergleichsbeispiel 3**

50 g (= 0,278 mol) Aeromyl 115 (physikalisch modifizierte, kaltwasserlösliche Stärke der Firma Südstärke; Restfeuchte = ca. 9,5 %) werden in 400 ml Wasser gelöst. Der pH wird mit 3 N NaOH auf 8 eingestellt und während der Reaktion konstant gehalten. Bei einer Reaktionstemperatur von 0 °C werden 27,27 g (= 0,278 mol) festes Maleinsäureanhydrid über einen Zeitraum von 2 h zugegeben. Es wird eine weitere Stunde gerührt, wobei die Reaktionsmischung auf Raumtemperatur gebracht wird. Anschließend wird noch 2 h bei 60°C nachreagiert. Der Substitutionsgrad (DS(NaOH)) beträgt 0,76.
Die Reaktionslösung wird einrotiert und im Vakuumtrockenschrank nachgetrocknet. Das Produkt wird gemahlen und nochmals 30 min bei 100°C im Vakuumtrockenschrank nachgetrocknet.
SRV = 12,6 g/g

**Vergleichsbeispiel 4**

50 g (= 0,278 mol) Aeromyl 115 (physikalisch modifizierte, kaltwasserlösliche Stärke der Firma Südstärke; Restfeuchte = ca. 9,5 %) werden in 400 ml Wasser gelöst. Der pH wird mit 3 N NaOH auf 8 eingestellt und während der Reaktion konstant gehalten. Es werden 13,6 mg Benzochinon zugegeben. Bei einer Reaktionstemperatur von 0 °C werden 27,27 g (= 0,278 mol) festes Maleinsäureanhydrid über einen Zeitraum von 2 h zugegeben. Es wird eine weitere Stunde gerührt, wobei die Reaktionsmischung auf Raumtemperatur gebracht wird (Nachreaktion). Der Substitutionsgrad (DS(NaOH)) beträgt 0,83.
Die Reaktionslösung wird einrotiert und im Vakuumtrockenschrank nachgetrocknet. Das Produkt wird gemahlen und nochmals 30 min bei 100°C im Vakuumtrockenschrank nachgetrocknet.
SRV = 19,4 g/g

**Alterung der Substanzen der Vergleichsbeispiele**

Auch die Messungen an den Produkten der Vergleichsbeispiele wurden nach Lagerung wiederholt (siehe Tabelle 3).

Tabelle 3

| VB | SRV Anfangswert | SRV nach (x) Tagen | Abweichung [%] |
|---|---|---|---|
| 1 | 14,3 | 10,7 (84) | -25 |
| 3 | 12,6 | 9,7 (84) | -23 |
| 4 | 19,4 | 10,0 (84) | -48 |

## Patentansprüche

1. Verfahren zur Herstellung von quellbaren, alterungsbeständigen Stärkemaleaten,
**dadurch gekennzeichnet,**
**daß** man ein quellbares Stärkemaleat mit einem oder mehreren ein- und/oder mehrfachfunktionellen SH-Gruppenhaltigen Nucleophil(en) nach Art einer Michael-Addition umsetzt.

2. Verfahren nach Anspruch 1,
**dadurch gekennzeichnet,**
**daß** ein quellbares Stärkemaleat verwendet wird, welches nach einem Verfahren erhältlich ist, bei dem Stärke oder modifizierte Stärke in einer einstufigen, wässrigen Reaktion mit Maleinsäureanhydrid oder einer Mischung von Carbonsäureanhydriden aufweisend Maleinsäureanhydrid, bei einem pH-Wert von 7 - 11 und einer Temperatur von 0 - 40 °C umgesetzt wird, wobei der pH-Wert durch Zugabe von wässriger Alkalilösung mit einer Konzentration

von ca. 10 - 50 Gew.-% im gewünschten Bereich gehalten wird.

3. Verfahren nach Anspruch 1, worin ein quellbares Stärkemaleat verwendet wird, welches nach einem Verfahren erhältlich ist, bei dem Stärke oder modifizierte Stärke mit Maleinsäureanhydrid oder einer Mischung von Carbonsäureanhydriden aufweisend Maleinsäureanhydrid in Gegenwart einer Base und ggf. eines Lösungsmittels umgesetzt wird, wobei man bei einer Temperatur von 80 - 180 °C für 10 min bis 10 h reagieren läßt, und das Lösungsmittel nur bis zu einem Gehalt von weniger als 100 Teile, bezogen auf 100 Teile Stärke, toleriert wird.

4. Verfahren nach Anspruch 3,
**dadurch gekennzeichnet,**
**daß** als Base Natriumcarbonat verwendet wird.

5. Verfahren nach Anspruch 1-3,
**dadurch gekennzeichnet,**
**daß** man als Nucleophil Mercaptoethanol einsetzt.

6. Verfahren nach Anspruch 1-3,
**dadurch gekennzeichnet,**
**daß** man als Nucleophil 1,2-Bis-(2-mercaptoethoxy)-ethan einsetzt.

7. Verfahren nach Anspruch 1-3,
**dadurch gekennzeichnet,**
**daß** man eine Mischung aus Mercaptoethanol und 1,2-Bis-(2-mercaptoethoxy)-ethan einsetzt.

8. Verfahren nach Anspruch 7,
**dadurch gekennzeichnet,**
**daß** man zwischen 0,001 und 0,5 eq Mercaptoethoxyethan in Kombination mit zwischen 0,5 und 0,999 eq Mercaptoethanol als Nucleophil einsetzt, wobei die Äquivalente sich auf die im Stärkemaleat enthaltenen Maleinsäureanhydrid-Mengen beziehen.

9. Verfahren nach einem oder mehreren der Ansprüche 1-3,
**dadurch gekennzeichnet,**
**daß** man als Nucleophil Pentaerythrit-tetrakis-(2-mercaptoacetat) einsetzt.

10. Verfahren nach einem oder mehreren der Ansprüche 1-3,
**dadurch gekennzeichnet,**
**daß** man eine Mischung aus Natriumbisulfit oder Natriumhydrogensulfit und Pentaerythrit-tetrakis-(2-mercaptoacetat) einsetzt.

11. Verfahren nach Anspruch 10,
**dadurch gekennzeichnet,**
**daß** man zwischen 0,001 und 0,5 eq Pentaerythrit-tetrakis-(2-mercapto-acetat) in Kombination mit zwischen 0,25 und 0,999 eq Natriumbisulfit als Nucleophil einsetzt, wobei die Äquivalente sich auf die im Stärkemaleat enthaltenen Maleinsäureanhydrid-Mengen beziehen.

12. Verfahren nach einem der vorhergehenden Ansprüche,
**dadurch gekennzeichnet,**
**daß** man die Umsetzung mit dem ein- und/oder mehrfachfunktionellen Nucleophil unmittelbar anschließend an die Synthese des Stärkemaleats in einer Eintopfreaktion bei Temperaturen zwischen Raumtemperatur und etwa 80 °C über einen Zeitraum von 10 min bis 24 h durchführt.

13. Verfahren nach einem oder mehreren der vorhergehenden Ansprüche,
**dadurch gekennzeichnet,**
**daß** man das quellbare Stärkemaleat so umsetzt, daß nur ein Teil seiner Doppelbindungen abreagiert, das so umgesetzte Stärkemaleat isoliert und dann in einem Lösungsmittel suspendiert und das suspendierte Stärkemaleat an seiner Oberfläche nachvernetzt.

14. Biologisch abbaubares Stärkemaleat erhältlich nach einem in den vorhergehenden Ansprüchen beschriebenen

Verfahren,
**gekennzeichnet durch**
eine Abnahme des im Teebeutel-Test ermittelten SRV (saline retention value) nach 100 Tagen von weniger als 20 %.

**15.** Stärkemaleat nach Anspruch 14,
**dadurch gekennzeichnet,**
**daß** die Abnahme des SRV nach 100 Tagen weniger als 10 % beträgt.

**16.** Verwendung des Stärkemaleats gemäß Anspruch 14 oder 15, in einer Menge von 100 Gew.-Teilen zusammen mit 0,7 - 70 Gew.-Teilen eines Antiblocking-Mittels auf Basis natürlicher oder synthetischer, bevorzugt hydrophiler Fasern oder Materialien mit großer Oberfläche als Superabsorber.

**17.** Verwendung des Stärkemaleats gemäß Anspruch 14 oder 15 als Superabsorber zusammen mit 1 - 5 Gew.-Teilen Kieselsäure oder Cellulosefasern als Antiblocking-Mittel.

**18.** Verwendung des Stärkemaleats gemäß einem der Ansprüche 14 oder 15 als Absorptionsmaterial zur Absorption von Wasser, wässrigen Lösungen, Dispersionen und Körperflüssigkeiten in Hygiene- und Tierhygiene, insbesondere in Windeln, Tampons und Inkontinenz-Produkten sowie in Verpackungsmaterialien für Fleisch und Fisch.

**19.** Verwendung des Stärkemaleats gemäß Anspruch 14 oder 15 als Absorptionsmaterial zur Absorption von Wasser und wässrigen Lösungen in Kulturgefäßen und zur Bodenverbesserung.

**20.** Verwendung des Stärkemaleats gemäß Anspruch 14 oder 15 als Absorptionsmaterial zur Absorption von Wasser und wässrigen Lösungen in Kabelummantelungen.

## Claims

**1.** A method of producing swellable, ageing-resistant starch maleates,
**characterised in that**
a swellable starch maleate is reacted, in the manner of a Michael addition, with one or more mono- and/or multifunctional nucleophiles which contain SH groups

**2.** A method according to claim 1,
**characterised in that**
a swellable starch maleate is used which is obtainable by a method in which starch or modified starch is reacted, in a single-stage aqueous reaction, with maleic anhydride or with maleic anhydride containing a mixture of carboxylic anhydrides at a pH of 7 - 11 and at a temperature of 0 - 40°C, wherein the pH is maintained within the desired range by the addition of an aqueous alkali solution with a concentration of about 10 - 50 % by weight.

**3.** A method according to claim 1, wherein a swellable starch material is used which is obtainable by a method in which starch or modified starch is reacted with maleic anhydride or with maleic anhydride containing a mixture of carboxylic anhydrides in the presence of a base and optionally of a solvent, wherein the batch is allowed to react at a temperature of 80 - 180 °C for 10 minutes to 10 hours, and the solvent is only tolerated up to a content of less than 100 parts with respect to 100 parts starch.

**4.** A method according to claim 3,
**characterised in that**
sodium carbonate is used as a base.

**5.** A method according to claims 1-3,
**characterised in that**
mercaptoethanol is used as a nucleophile.

**6.** A method according to claims 1-3,
**characterised in that**
1,2-bis-(2-mercaptoethoxy)-ethane is used as a nucleophile.

7. A method according to claims 1-3,
**characterised in that**
a mixture of mercaptoethanol and 1,2-bis-(2-mercaptoethoxy)-ethane is used.

8. A method according to claim 7,
**characterised in that**
between 0.001 and 0.5 equivalents of mercaptoethoxyethane in combination with between 0,.5 and 0.999 equivalents of mercaptoethanol are used as a nucleophile, wherein the equivalents relate to the amounts of maleic anhydride contained in the starch maleate.

9. A method according to one or more of claims 1-3,
**characterised in that**
pentaerythritol-tetrakis-(2-mercapto-acetate) is used as a nucleophile.

10. A method according to one or more of claims 1-3,
**characterised in that**
a mixture of sodium bisulphite or sodium hydrogen sulphite and pentaerythritol-tetrakis-(2-mercapto-acetate) is used.

11. A method according to claim 10,
**characterised in that**
between 0.001 and 0.5 equivalents of pentaerythritol-tetrakis-(2-mercapto-acetate) in combination with between 0.25 and 0.999 equivalents of sodium bisulphite are used as a nucleophile, wherein the equivalents relate to the amounts of maleic anhydride contained in the starch maleate.

12. A method according to any one of the preceding claims,
**characterised in that**
the reaction with the mono- and/or multi-functional nucleophile is conducted directly after the synthesis of the starch maleate, in a one-pot reaction at temperatures between room temperature and about 80°C over a period of 10 minutes to 24 hours.

13. A method according to one or more of the preceding claims,
**characterised in that**
the swellable starch maleate is reacted so that only a part of the double bonds thereof is reacted, the starch maleate which is reacted in this manner is isolated and is then suspended in a solvent and the suspended starch maleate is subsequently crosslinked at its surface.

14. A biodegradable starch maleate obtainable by a method described in the preceding claims,
**characterised by**
a decrease of less than 20 % in the SRV (saline retention value) after 100 days as determined by a tea bag test

15. A starch maleate according to claim 14,
**characterised in that**
the decrease in SRV after 100 days is less than 10 %.

16. Use of the starch maleate according to claims 14 or 15, in an amount of 100 parts by weight, together with 0.7 - 70 parts by weight of an anti-blocking agent based on natural or synthetic, preferably hydrophilic fibres or materials of large surface area, as a super-absorber.

17. Use of the starch maleate according to claims 14 or 15 as a super-absorber together with 1 - 5 parts by weight of hydrated silica or cellulose fibres as anti-blocking agents.

18. Use of the starch maleate according to either one of claims 14 or 15 as an absorption material for the absorption of water, aqueous solutions, dispersions and body fluids in hygiene and animal hygiene, particularly in bandages, tampons and incontinence products and in packaging materials for meat and fish.

19. Use of the starch maleate according to claims 14 or 15 as an absorption material for the absorption of water and aqueous solutions in culture vessels and for soil conditioning.

**20.** Use of the starch maleate according to claims 14 or 15 as an absorption material for the absorption of water and aqueous solutions in cable sheathings.

## Revendications

**1.** Procédé pour la préparation de maléates d'amidon gonflables, résistants au vieillissement,
**caractérisé en ce qu'**on transforme un maléate d'amidon gonflable avec un ou plusieurs nucléophile(s) contenant des groupes SH mono et/ou polyfonctionnels selon une addition de Michael.

**2.** Procédé selon la revendication 1,
**caractérisé en ce qu'**on utilise un maléate d'amidon gonflable qui peut être obtenu par un procédé selon lequel de l'amidon ou de l'amidon modifié est transformé, au cours d'une réaction aqueuse, en une seule étape, avec de l'anhydride maléique ou un mélange d'anhydrides carboxyliques comportant de l'anhydride maléique, à un pH de 7-11 et à une température de 0-40°C, dans lequel le pH est maintenu dans la plage souhaitée par addition de solution alcaline aqueuse à une concentration d'environ 10-50% en poids.

**3.** Procédé selon la revendication 1, dans lequel on utilise un maléate d'amidon gonflable qui peut être obtenu par un procédé selon lequel de l'amidon ou de l'amidon modifié est transformé avec de l'anhydride maléique ou un mélange d'anhydrides carboxyliques comportant de l'anhydride maléique en présence d'un base et, éventuellement, d'un solvant, dans lequel la réaction a lieu à une température de 80 - 180 °C pendant 10 minutes à 10 heures, et le solvant n'est toléré que jusqu'à une teneur de moins de 100 parties par rapport à 100 parties d'amidon.

**4.** Procédé selon la revendication 3, **caractérisé en ce que** du carbonate de sodium est utilisé comme base.

**5.** Procédé selon les revendications 1 à 3,
**caractérisé en ce que** du mercaptoéthanol est utilisé comme nucléophile.

**6.** Procédé selon les revendications 1 à 3,
**caractérisé en ce que** du 1,2-bis-(2-mercaptoéthoxy)-éthane est utilisé comme nucléophile.

**7.** Procédé selon les revendications 1 à 3,
**caractérisé en ce qu'**un mélange de mercaptoéthanol et de 1,2-bis-(2-mercaptoéthoxy)-éthane est utilisé.

**8.** Procédé selon la revendication 7,
**caractérisé en ce qu'**entre 0,001 et 0,5 eq de mercaptoéthoxyéthane en combinaison avec entre 0,5 et 0,999 eq de mercaptoéthanol sont utilisés comme nucléophile, les équivalents se rapportant aux quantités d'anhydride maléique contenues dans le maléate d'amidon.

**9.** Procédé selon une ou plusieurs des revendications 1 à 3,
**caractérisé en ce que** du tétrakis-(2-mercapto-acétate) de pentaérythritol est utilisé comme nucléophile.

**10.** Procédé selon une ou plusieurs des revendications 1 à 3,
**caractérisé en ce qu'**un mélange de bisulfite de sodium, ou hydrogénosulfite de sodium, et de tétrakis-(2-mercapto-acétate) de pentaérythritol est utilisé.

**11.** Procédé selon la revendication 10,
**caractérisé en ce qu'**entre 0,01 et 0,5 eq de tétrakis-(2-mercapto-acétate) de pentaérythritol en combinaison avec entre 0,25 et 0,999 eq de bisulfite de sodium sont utilisés comme nucléophile, les équivalents se rapportant aux quantités d'anhydride maléique contenues dans le maléate d'amidon.

**12.** Procédé selon l'une quelconque des revendications précédentes,
**caractérisé en ce que** la réaction avec le nucléophile mono et/ou polyfonctionnel est effectuée immédiatement à la suite de la synthèse du maléate d'amidon au cours d'une réaction dans un seul récipient à des températures entre la température ambiante et environ 80°C sur une période de 10 minutes à 24 heures.

**13.** Procédé selon une ou plusieurs des revendications précédentes,
**caractérisé en ce qu'**on transforme le maléate d'amidon gonflable de telle sorte qu'une partie seulement

de ses doubles liaisons réagit, on isole le maléate d'amidon ayant ainsi réagi puis on le met en suspension dans un solvant et on post-réticule à sa surface le maléate d'amidon en suspension.

**14.** Maléate d'amidon biodégradable obtenu selon l'un des procédés décrits dans les revendications précédentes,
**caractérisé en ce que** la diminution de l'IRS (indice de rétention saline), déterminé au cours du test du sachet de thé au bout de 100 jours, est inférieure à 20%.

**15.** Maléate d'amidon selon la revendication 14,
**caractérisé en ce que** la diminution de l'IRS au bout de 100 jours est inférieure à 10%.

**16.** Utilisation du maléate d'amidon selon la revendication 14 ou 15 en une quantité de 100 parties en poids avec 0,7-70 parties en poids d'un agent anti-blocage à base de fibres ou de matières naturelles ou synthétiques, de préférence hydrophiles, présentant une grande surface, comme super-absorbant.

**17.** Utilisation du maléate d'amidon selon la revendication 14 ou 15 comme super-absorbant avec 1-5 parties en poids d'acide silicique ou de fibres de cellulose comme agent anti-blocage.

**18.** Utilisation du maléate d'amidon selon l'une quelconque des revendications 14 ou 15 comme matière absorbante pour l'absorption d'eau, de solutions aqueuses, de dispersions et de liquides corporels dans le domaine de l'hygiène et de l'hygiène animale, en particulier dans les couches, les tampons, les produits contre l'incontinence ainsi que dans les matériaux d'emballage pour la viande et le poisson.

**19.** Utilisation du maléate d'amidon selon la revendication 14 ou 15, comme matière absorbante pour l'absorption d'eau et de solutions aqueuses dans des récipients de culture et pour l'amélioration des sols.

**20.** Utilisation du maléate d'amidon selon la revendication 14 ou 15 comme matière absorbante pour l'absorption d'eau et de solutions aqueuses dans des gaines de câbles.